(19) **Europäisches Patentamt**
**European Patent Office**
**Office européen des brevets**

(11) **EP 4 180 730 B1**

(12) **EUROPEAN PATENT SPECIFICATION**

(45) Date of publication and mention
of the grant of the patent:
**24.07.2024   Bulletin 2024/30**

(21) Application number: **22206128.5**

(22) Date of filing: **08.11.2022**

(51) International Patent Classification (IPC):
*F24F 7/003* (2021.01)      *F24F 7/06* (2006.01)
*F24F 8/22* (2021.01)       *F24F 13/02* (2006.01)
*F24F 13/06* (2006.01)      *F24F 13/08* (2006.01)
*B01D 53/88* (2006.01)      *A61L 9/20* (2006.01)

(52) Cooperative Patent Classification (CPC):
**F24F 13/02; A61L 9/20; B01D 53/007; B01D 53/44;**
**F24F 7/003; F24F 7/065; F24F 8/22; F24F 13/06;**
**F24F 13/084;** A61L 2209/135; A61L 2209/16;
B01D 2257/91; B01D 2258/06; B01D 2259/4508;
B01D 2259/804

(54) **AIR PASSAGE DEVICE, STERILIZATION SYSTEM, AND DUCTING SYSTEM**

LUFTDURCHLASSVORRICHTUNG, STERILISATIONSSYSTEM UND LEITUNGSSYSTEM

DISPOSITIF DE PASSAGE D'AIR, SYSTÈME DE STÉRILISATION ET SYSTÈME DE CANALISATION

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **11.11.2021   IT 202100028724**

(43) Date of publication of application:
**17.05.2023   Bulletin 2023/20**

(73) Proprietor: **Russo, Dario**
**8802 Kilchberg (CH)**

(72) Inventor: **Russo, Dario**
**8802 Kilchberg (CH)**

(74) Representative: **Gamba, Alessandro**
**Jacobacci & Partners S.p.A.**
**Piazza della Vittoria, 11**
**25122 Brescia (IT)**

(56) References cited:
**WO-A1-2011/135601      WO-A1-2016/178698**
**US-A1- 2003 230 477      US-A1- 2004 018 125**

**Description**

<u>Field of application</u>

The present invention relates to an air passage device suitable for allowing the passage of air while preventing the passage of light, a sterilization system, and a ducting system.

In the prior art, there are situations where it is necessary to facilitate the passage of air without allowing the passage of light, from an outdoor environment to a closed environment, or vice versa, or between two closed environments.

For example, some closed environments need to be ventilated by preventing the entry of sunlight, such as darkrooms or special analysis laboratories, or greenhouses for growing certain plants.

Alternatively, in some situations it is necessary to shield light rays that are used for air treatment or therapy applications, so that they do not escape from the chamber in which they are emitted, while still allowing air exchange.

Disadvantageously, the known solutions for this requirement have drawbacks.

The most common solutions involve the use of a complex set of intricate channels and filters, so that light is absorbed through numerous reflections in said channels while at the same time allowing a portion of air to pass through. Document WO2016/178698A discloses an active oxidation and purifying system, provided to increase or maximize the rate of photocatalytic oxidation and ambient air purification capacity by implementing both direct ultraviolet (UV) light and reflected UV light directed to the surface and apertures of active cell panels coated with a photocatalytic material. A first set of the apertures could be disposed about 45 degrees relative to a median axis along the first and second surfaces, while a second set of apertures could be disposed about negative 45 degrees relative to the same median axis in order to increase the surface area impinged by the direct and reflected UV light.

Disadvantageously, these solutions force the flow of air into the channels or through the filter fibers, changing its direction in a sudden and abrupt way. Because of these changes, turbulence and a multiplicity of boundary layers are generated in the airflow in the areas near the walls of channels or between the filter cavities. This turbulence leads to an amplification of noise in the ventilation process, as well as an increase in the resistance that air must overcome to pass through ventilation ducts.

Furthermore, disadvantageously, these known solutions require the use of very powerful fans, and consequently result in an additional increase in power consumption.

In other words, the known system solutions belonging to the prior art fail to effectively perform both functions: they either perform efficient air passage or efficient light blocking.

In other words, some systems are very efficient in terms of ventilation but do not block all the light, while other systems block a large percentage of light rays but allow only a weakened airflow.

<u>Solution of the invention</u>

Therefore, there appears to be a strong need to provide an air passage device that is able to overcome the aforementioned drawbacks of the prior art.

This requirement is met by an air passage device defined in claim 1.

The claims dependent thereon describe preferred or advantageous embodiments of the invention, comprising further advantageous features.

<u>Description of the drawings</u>

The features and advantages of the air passage device, of a sterilization system and of a ducting system will become apparent from the following description of a number of preferred embodiments, given by way of non-limiting example, with reference to the attached figures, in which:

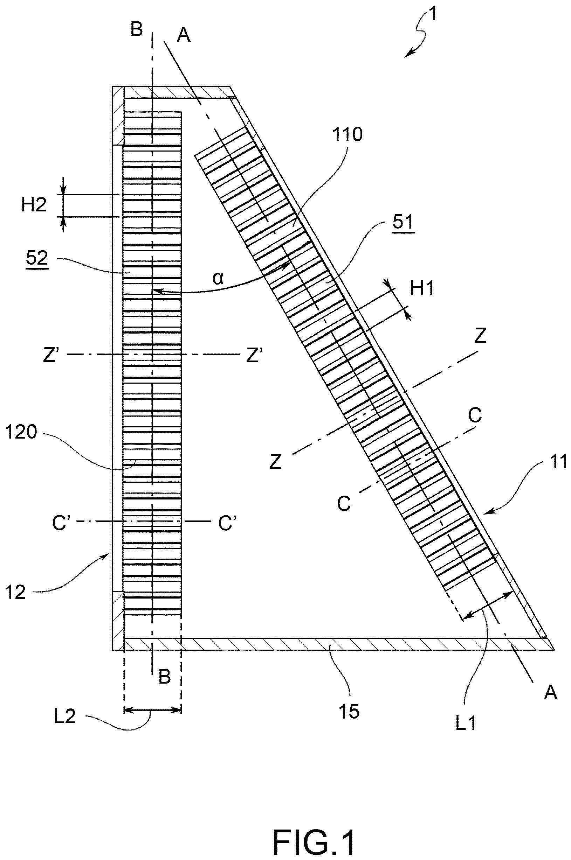

- Fig. 1 is a sectional side view of an air passage device in accordance with a preferred embodiment according to the present invention;
- Fig. 2a and 2b are front views of two preferred alternative embodiments of the present invention;
- Fig. 3 is a sectional side view of an air passage device in accordance with a preferred embodiment, according to the present invention;
- Fig. 3b is a front view of the air passage device in Fig. 3a;

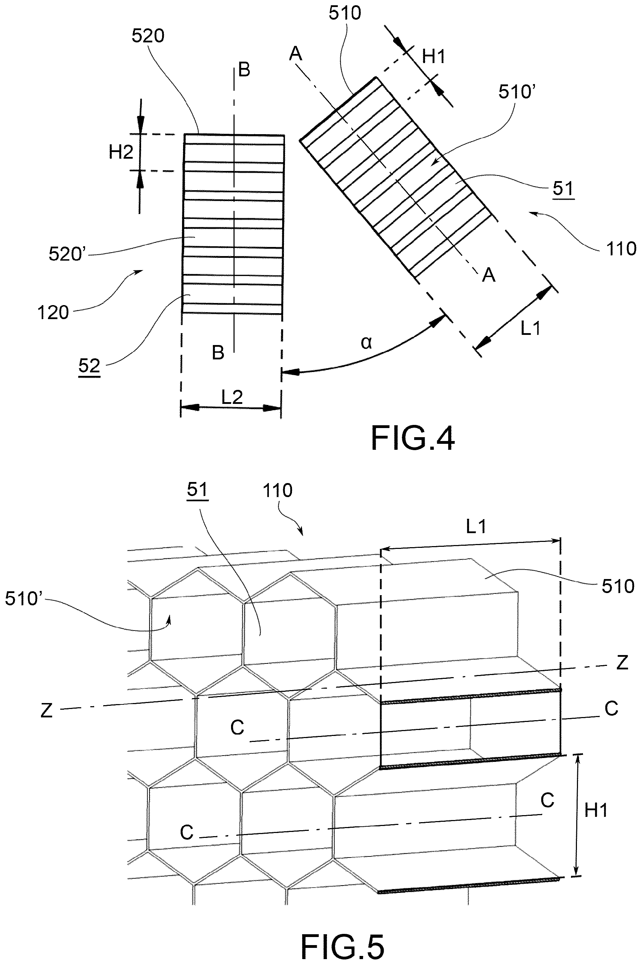

- Fig. 4 is a detailed side view of the grid elements of an air passage device in accordance with a preferred embodiment, according to the present invention;
- Fig. 5 is an axonometric view of a detail of a grid element of an air passage device in accordance with a preferred embodiment according to the present invention.

- Fig. 6 is a schematic side view in which the path taken by light rays inside an air passage device is schematized in accordance with an embodiment according to the present invention;
- Fig. 7 shows a perspective view of an air passage device in accordance with an embodiment according to the present invention;
- Fig. 8 is a longitudinal section of a sterilization system in accordance with a preferred embodiment according to the present invention.

Detailed description

With reference to the aforesaid figures, an air passage device for air ventilation in closed environments has been referred to collectively with reference number 1.

In the present discussion, speaking of "closed environments" refers to the rooms of a building, and also to the inner chambers, ducts, and compartments of a device or apparatus or system, such as the irradiation chamber of a sterilization system. For example, the sterilization system 9 that comprises an air passage device 1 is also an object of the present invention.

According to the invention, the air passage device comprises a first grid element 11 which extends mainly on a first imaginary plane A and which has a first thickness L1 along a first air passage direction Z-Z perpendicular to said first imaginary plane A.

Furthermore, the first grid element 2 comprises a first grid body 110 in which first openings 51 are obtained, each extending respectively for the entire first thickness L1 with a first opening height H1, along first main opening axes C-C substantially parallel respectively to the first air passage direction Z-Z.

Moreover, according to the present invention, said first openings 51 are delimited by first side walls 510 having a first diffusive reflecting inner surface 510'.

According to the present invention, moreover, the air passage device comprises a second grid element 12 which extends on a second imaginary plane B and which has a second thickness L2 along a second air passage direction Z'-Z' perpendicular to said second imaginary plane B.

Furthermore, the second grid element 12 comprises a second grid body 120 in which second openings 52 are obtained, each extending respectively for the entire second thickness L2 with a second opening height H1, along second main opening axes C'-C' substantially parallel respectively to the second air passage direction Z'-Z'.

Moreover, according to the present invention, said second openings 52 are delimited by second side walls 520 having a second diffusive reflecting inner surface 520'.

As just described in the aforesaid paragraphs, "first" denotes features and components of the first grid element 11, and "second" denotes features and components of the second grid element 12. This distinction is only a convention and does not indicate differences in magnitude or flow.

Preferably, said first and second diffusive reflecting inner surfaces 510' and 520' are made of a material having a total reflectance coefficient lower than 0.5, preferably lower than 0.2 in the light ray spectrum of interest. Total reflectance is defined as the union of diffusive reflectance and specular reflectance.

According to the invention, the air passage device further comprises a support frame 15 that supports and connects said first grid element 11 and said second grid element 12, so that the first grid element 11 and the second grid element 12 are crossed in series by air.

Specifically, according to the invention, the first imaginary plane A and the second imaginary plane B are mutually incident with an inclination angle $\alpha$ that is a function of said first and second thicknesses L1, L2 and of said first and second opening heights H1, H2, so that at least one portion of a light ray entering said openings 51, 52 is absorbed by the diffusive reflecting inner surface 510', 520' of at least one of said openings 51, 52.

Preferably, the inclination angle $\alpha$ is a function of said first and second thicknesses L1, L2 and of said first and second opening heights H1, H2 according to the formula:

$$\alpha \geq \text{arctan } (H1/L1) + \text{arctan } (H2/L2)$$

In other words, the inclination angle is greater than or equal to the sum of the arctangent of the ratio H1/L1 plus the arctangent of the ratio H2/L2.

According to a preferred embodiment, the air passage device comprises a minimum number of grid elements that is a function of the number of reflections of the light rays desired.

In particular, preferably, the minimum number of grid elements varies according to the formula:

$$\text{Nmin = Nref + 1}$$

where Nmin is the minimum total number of grid elements and Nref is the minimum number of reflections to be obtained.

In other words, for example, if at least two reflections are to be obtained, the air passage device must comprise three grid elements mutually inclined at an inclination angle $\alpha$.

It is clear that the inclination angle $\alpha$ is variable for each pair of mutually inclined grid elements, and varies with the geometry and dimensions of the respective grid elements.

Preferably, moreover, the first opening height H1 is less, preferably half, with respect to said first thickness L1 and the second opening height H2 is less, preferably half, with respect to said second thickness L2.

Even more preferably, the first opening height H1 is lower than half of said first thickness L1 and the second opening height H2 is lower than half of said second thickness L2.

In an advantageous embodiment, the first thickness L1 coincides with the second thickness L2.

In one embodiment of the invention, the air passage device 1 comprises an auxiliary grid element 16 arranged in series with said first grid element 11 and second grid element 12.

According to one embodiment of the invention, the auxiliary grid element 16 also comprises a third grid body in which third openings are obtained.

Preferably, this auxiliary grid element 16 extends along a third imaginary plane D mutually incident with said first and second imaginary planes A, B.

Preferably, moreover, said third imaginary plane D forms, with the first imaginary plane A, a first auxiliary inclination angle $\delta$ and forms, with the second imaginary plane B, a second auxiliary inclination angle $\beta$.

Preferably, said auxiliary grid element 16 is interposed between said first grid element 11 and said second grid element 12.

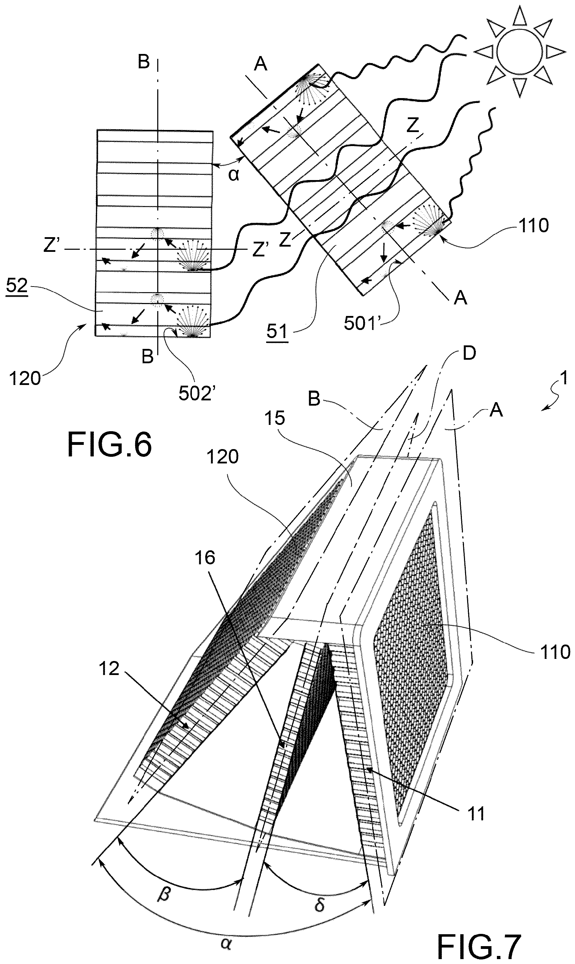

According to a preferred embodiment, with reference to an example embodiment depicted in Fig. 7, the sum of these first and second auxiliary inclination angles $\delta$ and $\beta$ coincides with the inclination angle $\alpha$.

In an alternative embodiment according to the present invention, the first auxiliary inclination angle $\delta$ and/or the second auxiliary inclination angle $\beta$ coincides with the inclination angle $\alpha$.

Preferably, the air passage device 1 comprises a plurality of auxiliary grid elements.

According to one embodiment, in accordance with the present invention, the air passage device comprises a total number Ntot of grid elements (including said first and second grid elements and said auxiliary grid elements) at least greater than or equal to said minimum number of grid elements Nmin.

Preferably, in an air passage device 1 comprising a total number Ntot of grid elements, at least a minimum number of grid elements Nmin, even if not adjacent, must be mutually inclined with said inclination angle $\alpha$.

In other words, by way of example, in an air passage device comprising a total number of grid elements Ntot equal to five, and where the number of desired reflections Nref is equal to one, the minimum number of grid elements is equal to two. Therefore, preferably, the air passage device 1 comprises at least two grid elements inclined to each other by an inclination angle $\alpha$ that follows the formula described above.

Preferably, the additional grid elements 16 mutually form angles other than said inclination angle $\alpha$.

According to a preferred embodiment, said first openings 51 and said second openings 52 have polygonal cross-sections.

Preferably, said first and second openings 51 and 52 have hexagonal cross-sections.

Even more preferably, the first diffusive reflecting inner surface 510' and the second diffusive reflecting inner surface 520' are made of a material that reflects the light rays diffusively in the spectrum of interest. In other words, they are made of a Lambertian-type material, having features of a non-ideal diffuser.

It is specified that a non-ideal diffuser means a material suitable for absorbing a portion of the incident ray, and reflecting the remaining portion diffusively.

In other words, if the light rays belong to the visible spectrum, said first and second diffusive reflecting inner surfaces 510' and 520' are made of a material having a total reflectance coefficient (diffuse and specular) lower than 0.5, preferably lower than 0.2, in the visible spectrum.

Alternatively, if the light rays belong to the UV spectrum, said first and second diffusive reflecting inner surfaces 510' and 520' are made of a material having a total reflectance coefficient (diffuse and specular) lower than 0.5, preferably lower than 0.2, in the UV spectrum.

In a variant embodiment, the air passage device also comprises a cover panel 125 applied externally to the first grid element 11.

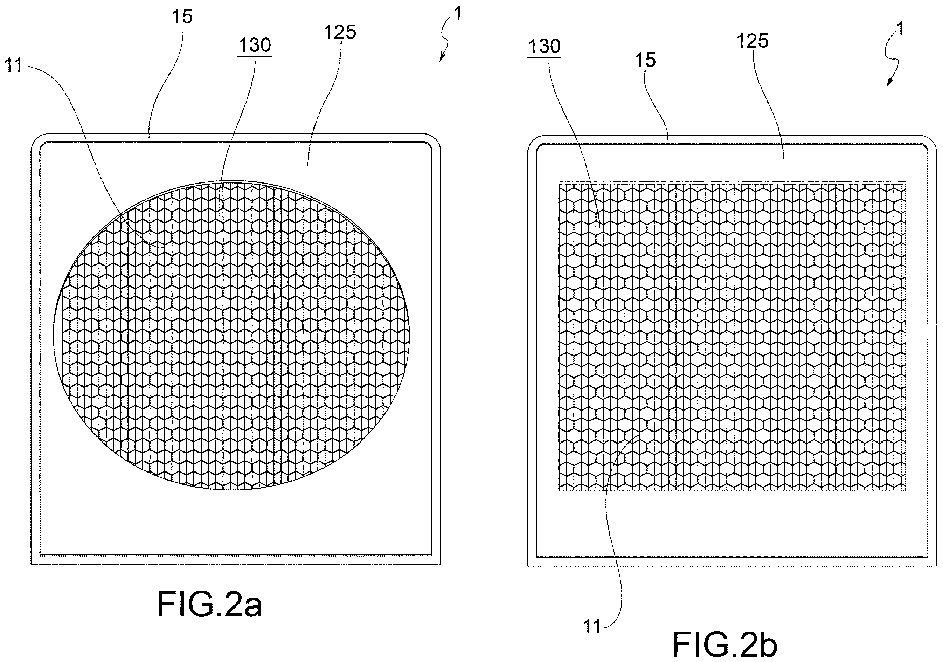

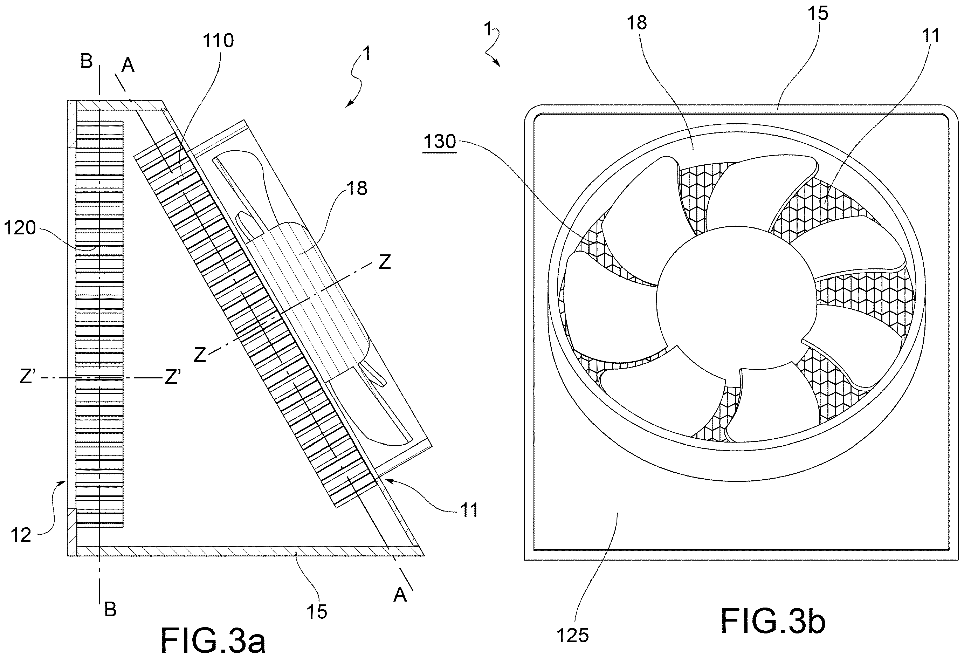

Preferably, with reference to some of the example embodiments depicted in Fig. 2a, 2b, 3a and 3b, a window 130 is obtained in this cover panel 125, which window provides access to the first grid body 110 of said first grid element 11.

According to a preferred embodiment, this window 130 has a polygonal shape, for example, it is a rectangle, or it has an elliptical shape, or it is circular.

According to one embodiment, the air passage device comprises a cover panel 125 also applied externally to the second grid element 12.

According to a variant embodiment, the air passage device 1 comprises a fan 18 inside the first grid element 11, for the suction of the air passing through said first grid element 11.

According to a further variant embodiment, the fan 18 is inside the second grid element 12, for the purpose of suctioning the air passing through said second grid element 12.

Preferably, with reference to an example embodiment depicted in Fig. 3a and 3b, said fan 18 is positioned at said window 130.

As mentioned above, a sterilization system 9 for sterilizing air in a closed environment also forms an object of the present invention.

According to the present invention, the sterilization system 9 comprises:

- an irradiation chamber 90 extending along an axis X-X, between an air inlet end 91 and an air outlet end 92, wherein said irradiation chamber 90 is peripherally delimited by a casing 900 having a reflective casing inner surface;
- an inlet mouth 910 in proximity to said air inlet end 91 and an outlet mouth 920 in proximity to said air outlet end 92;

- an emitter 95 suitable for emitting UV rays into the irradiation chamber 90;
- an air passage device 1 according to the present invention and as described.

Preferably, the air passage device 1 is placed in proximity to said outlet mouth 920, so that said air passage device 1 blocks the exit of UV rays from the sterilization system 9.

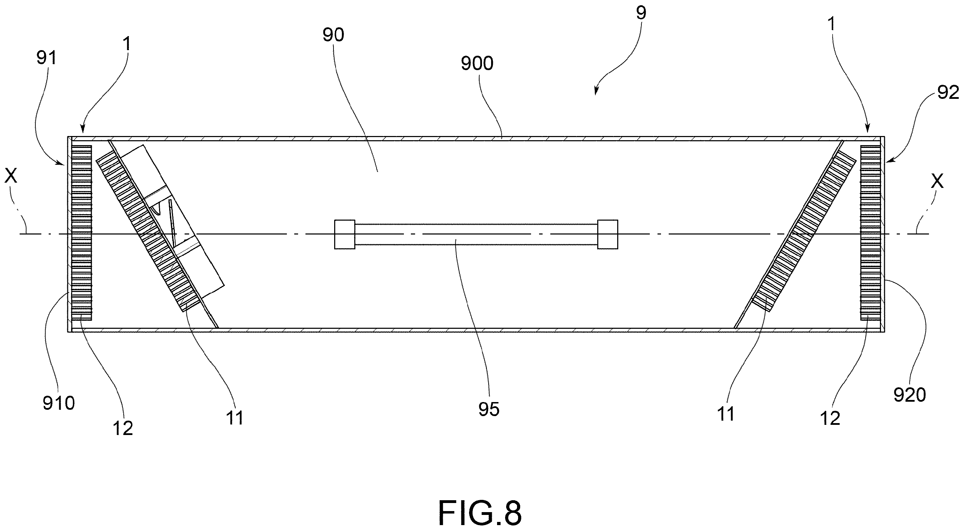

Preferably, with reference to an example shown in Fig. 8, this sterilization system 9 comprises an air passage device 1 according to the invention also in proximity to the inlet mouth 910.

It is also a subject of the present invention to provide a ducting system for a closed environment, comprising an air passage device 1 according to the claims 1 to 7.

This ducting system is suitable for allowing the ventilation of the closed environment by preventing the passage of light rays to or from said closed environment, preferably by preventing the entry of light rays from the ducting system.

Preferably, this closed environment is a dark environment, such as a darkroom.

Innovatively, the air passage device, the sterilization system and the ducting system covered by the present invention largely fulfill the intended object, overcoming the typical problems of the prior art.

Advantageously, the air passage device promotes air flow, minimizing the exit of light rays.

Advantageously, the air passage device is easy to assemble and arrange.

Advantageously, the air passage device may be easily adapted to any sterilization system or any pre-existing ducting system.

Advantageously, the air passage device also does not require the use of filters.

Advantageously, the air passage device does not force abrupt changes in flow, solving the problem of air pressure drops.

Advantageously, the air passage device according to the invention minimizes the turbulence, noise and air flow resistance caused when blocking the passage of light.

Advantageously, the grid elements made of a material having a total reflectance coefficient lower than 0.5, preferably lower than 0.2, and preferably with the features of a non-ideal diffuser, allow for a good part of the reflected portion of the light ray to be absorbed with each reflection.

Advantageously, using a fan positioned externally to the first or second grid element allows for increased airflow through the air passage device.

Advantageously, the sterilization system according to the present invention effectively blocks the exit of UV rays from the irradiation chamber.

Advantageously, the ducting system according to the present invention is effectively applicable to the walls of darkrooms or places that need to maintain a dark condition by blocking the entry of light but allowing the recirculation of air.

It is clear that a person skilled in the art may make changes to the invention in order to meet contingent needs, as long as said changes are all falling within the scope of protection as defined in the following claims.

List of reference numbers:

1 air passage device
11 first grid element
12 second grid element
110 first grid body
120 second grid body
A first imaginary plane

B second imaginary plane
C third imaginary plane
L1 first thickness
L2 second thickness
H1 first opening height
H2 second opening height
Z-Z first air passage direction
Z'-Z' second air passage direction
15 support frame
18 fan
125 cover panel
130 window
51 first openings
52 second openings
510 first side walls
520 second side walls
510' first diffusive reflecting inner surface
520' second diffusive reflecting inner surface
C-C first main opening axes
C'-C' second main opening axes
$\alpha$ inclination angle
$\delta$ first auxiliary inclination angle
$\beta$ second auxiliary inclination angle
16 auxiliary grid element
9 sterilization system
90 irradiation chamber
91 air inlet end
92 air outlet end
910 inlet mouth
920 outlet mouth
900 casing
95 emitter
X-X axis

**Claims**

1. An air passage device (1) for the ventilation of air in closed environments, comprising:

 - a first grid element (11) extending mainly on a first imaginary plane (A) and which has a first thickness (L1) along a first air passage direction (Z-Z) perpendicular to said first imaginary plane (A), wherein said first grid element (11) comprises a first grid body (110) in which first openings (51) are obtained, each extending respectively for the entire first thickness (L1) with a first opening height (H1), along main opening axes (C-C) which are substantially parallel respectively to the first air passage direction (Z-Z), wherein said first openings (51) are delimited by first side walls (510) having a first diffusive reflecting inner surface (510');
 - a second grid element (12) extending on a second imaginary plane (B) and which has a second thickness (L2) along a second air passage direction (Z'-Z') which is perpendicular to said second imaginary plane (B), wherein said second grid element (12) comprises a second grid body (120) in which second openings (52) are obtained each extending respectively for the entire second thickness (L2) with a second opening height (H2), along second main opening axes (C'-C') which are substantially parallel respectively to the second air passage direction (Z'-Z'), wherein said second openings (52) are delimited by second side walls (520) having a second diffusive reflecting inner surface (520');
 - a support frame (15) which supports and connects said first grid element (11) and said second grid element (12), the air passage device being configured so that the first grid element (11) and the second grid element (12) are crossable in series by air, wherein the first imaginary plane (A) and the second imaginary plane (B) are mutually incident with an inclination angle ($\alpha$) which is a function of said first and second thicknesses (L1, L2) and of said first and second opening heights (H1, H2), so that at least one portion of a light ray entering said openings (51, 52) is absorbed by the diffusive reflecting inner surface (510', 520') of at least one of said openings (51, 52).

2. Air passage device (1) according to claim 1, wherein the first opening height (H1) is less, preferably about half, with respect to said first thickness (L1) and the second opening height (H2) is less, preferably about half, with respect to said second thickness (L2).

3. Air passage device (1) according to any one of the preceding claims, wherein the first thickness (L1) coincides with the second thickness (L2).

4. Air passage device (1) according to any one of the preceding claims, comprising at least one auxiliary grid element (16) arranged in series with said first grid element (11) and second grid element (12).

5. Air passage device (1) according to any one of the preceding claims, wherein the first diffusive reflecting inner surface (510') and the second diffusive reflecting inner surface (520') are made of a material having a total reflectance coefficient lower than 0.5, preferably lower than 0.2 in the light ray spectrum of interest.

6. Air passage device (1) according to any one of the preceding claims, wherein the first diffusive reflecting inner surface (510') and the second diffusive reflecting inner surface (520') are made of a material which reflects the light rays diffusively.

7. Air passage device (1) according to any one of the preceding claims, comprising a fan (18) inside the first grid element (11) which is configured to suction the air passing through said first grid element (11).

8. A sterilization system (9) for sterilizing air in a closed environment, comprising:

- an irradiation chamber (90) extending along an axis (X-X), between an air inlet end (91) and an air outlet end (92), wherein said irradiation chamber (90) is peripherally delimited by a casing (900) having a reflective casing inner surface,
- an inlet mouth (910) in proximity to said air inlet end (91) and an outlet mouth (920) in proximity to said air outlet end (92);
- an emitter (95) suitable for emitting UV rays into the irradiation chamber (90);
- an air passage device (1) according to any one of the preceding claims; wherein said air passage device (1) is positioned in proximity to said outlet mouth (920) and configured to block an exit of UV rays from the sterilization system (9).

9. Sterilization system (9) according to claim 8, further comprising an air passage device (1) according to any one of the preceding claims 1 to 7 positioned in proximity to the inlet mouth (910).

10. A ducting system for a closed environment, comprising an air passage device (1) according to any one of claims 1 to 7, wherein said ducting system is configured to allow the ventilation of the closed environment preventing the passage of light rays from or towards said closed environment.


**Patentansprüche**

1. Luft-Durchgangsvorrichtung (1) für die Ventilation von Luft in geschlossenen Umgebungen, umfassend:

- ein erstes Gitterelement (11), welches sich hauptsächlich an einer ersten imaginären Ebene (A) erstreckt und welches eine erste Dicke (L1) entlang einer ersten Luft-Durchgangsrichtung (Z-Z) senkrecht zu der ersten imaginären Ebene (A) aufweist, wobei das erste Gitterelement (11) einen ersten Gitterkörper (110) umfasst, in welchem erste Öffnungen (51) erhalten sind, von welchen sich jede entsprechend für die gesamte erste Dicke (L1) mit einer ersten Öffnungshöhe (H1) entlang Haupt-Öffnungsachsen (C-C) erstreckt, welche jeweils im Wesentlichen parallel zu der ersten Luft-Durchgangsrichtung (Z-Z) sind, wobei die ersten Öffnungen (51) durch erste Seitenwände (510) begrenzt sind, welche eine erste diffusiv reflektierende Innenfläche (510') aufweisen;
- ein zweites Gitterelement (12), welches sich an einer zweiten imaginären Ebene (B) erstreckt und welches eine zweite Dicke (L2) entlang einer zweiten Luft-Durchgangsrichtung (Z'-Z') aufweist, welche senkrecht zu der zweiten imaginären Ebene (B) ist, wobei das zweite Gitterelement (12) einen zweiten Gitterkörper (120) umfasst, in welchem zweite Öffnungen (52) erhalten sind, von welchen sich jede entsprechend für die gesamte zweite Dicke (L2) mit einer zweiten Öffnungshöhe (H2) entlang zweiten Haupt-Öffnungsachsen (C'-C') erstreckt, welche jeweils im Wesentlichen parallel zu der zweiten Luft-Durchgangsrichtung (Z'-Z') sind, wobei die zweiten Öffnungen (52) durch zweite Seitenwände (520) begrenzt sind, welche eine zweite diffusiv reflektierende Innenfläche (520') aufweisen;

- einen Tragerahmen (15), welcher das erste Gitterelement (11) und das zweite Gitterelement (12) trägt und verbindet, wobei die Luft-Durchgangsvorrichtung derart eingerichtet ist, dass das erste Gitterelement (11) und das zweite Gitterelement (12) in Reihe von Luft durchkreuzbar sind, wobei die erste imaginäre Ebene (A) und die zweite imaginäre Ebene (B) wechselseitig mit einem Neigungswinkel ($\alpha$) einfallend sind, welcher eine Funktion der ersten und zweiten Dicken (L1, L2) und der ersten und zweiten Öffnungshöhen (H1, H2) ist, so dass wenigstens ein Teil eines Lichtstrahls, welcher in die Öffnungen (51, 52) eintritt, durch die diffusiv reflektierende Innenfläche (510', 520') von wenigstens einer der Öffnungen (51, 52) absorbiert wird.

2. Luft-Durchgangsvorrichtung (1) nach Anspruch 1, wobei die erste Öffnungshöhe (H1) weniger, vorzugsweise etwa die Hälfte, bezogen auf die erste Dicke (L1) ist, und die zweite Öffnungshöhe (H2) weniger, vorzugsweise etwa die Hälfte, bezogen auf die zweite Dicke (L2) ist.

3. Luft-Durchgangsvorrichtung (1) nach einem der vorhergehenden Ansprüche, wobei die erste Dicke (L1) mit der zweiten Dicke (L2) zusammenfällt.

4. Luft-Durchgangsvorrichtung (1) nach einem der vorhergehenden Ansprüche, umfassend wenigstens ein Hilfsgitterelement (16), welches in Reihe mit dem ersten Gitterelement (11) und dem zweiten Gitterelement (12) angeordnet ist.

5. Luft-Durchgangsvorrichtung (1) nach einem der vorhergehenden Ansprüche, wobei die erste diffusiv reflektierende Innenfläche (510') und die zweite diffusiv reflektierende Innenfläche (520') aus einem Material hergestellt sind, welches einen Gesamt-Reflexionskoeffizienten kleiner als 0,5, vorzugsweise kleiner als 0,2, in dem Lichtstrahl-Spektrum von Interesse aufweist.

6. Luft-Durchgangsvorrichtung (1) nach einem der vorhergehenden Ansprüche, wobei die erste diffusiv reflektierende Innenfläche (510') und die zweite diffusiv reflektierende Innenfläche (520') aus einem Material hergestellt sind, welches die Lichtstrahlen diffus reflektiert.

7. Luft-Durchgangsvorrichtung (1) nach einem der vorhergehenden Ansprüche, umfassend ein Gebläse (18) innerhalb des ersten Gitterelements (11), welches dazu eingerichtet ist, die durch das erste Gitterelement (11) hindurchtretende Luft anzusaugen.

8. Sterilisierungssystem (9) zum Sterilisieren von Luft in einer geschlossenen Umgebung, umfassend:

- eine Bestrahlungskammer (90), welche sich entlang einer Achse (X-X) zwischen einem Luft-Einlassende (91) und einem Luft-Auslassende (92) erstreckt, wobei die Bestrahlungskammer (90) umfänglich durch ein Gehäuse (900) begrenzt ist, welches eine reflektierende Gehäuse-Innenfläche aufweist,
- eine Einlassmündung (910) in der Nähe des Luft-Einlassendes (91) und eine Auslassmündung (920) in der Nähe des Luft-Auslassendes (92);
- einen Emitter (95), welcher geeignet ist, UV-Strahlen in die Bestrahlungskammer (90) zu emittieren;
- eine Luft-Durchgangsvorrichtung (1) nach einem der vorhergehenden Ansprüche; wobei die Luft-Durchgangsvorrichtung (1) in der Nähe der Auslassmündung (920) positioniert und dazu eingerichtet ist, ein Austreten von UV-Strahlen aus dem Sterilisierungssystem (9) zu blockieren.

9. Sterilisierungssystem (9) nach Anspruch 8, ferner umfassend eine Luft-Durchgangsvorrichtung (1) nach einem der Ansprüche 1 bis 7, welche in der Nähe der Einlassmündung (910) positioniert ist.

10. Leitungssystem für eine geschlossene Umgebung, umfassend eine Luft-Durchgangsvorrichtung (1) nach einem der Ansprüche 1 bis 7, wobei das Leitungssystem dazu eingerichtet ist, die Ventilation der geschlossenen Umgebung zu erlauben, wobei der Durchgang von Lichtstrahlen aus oder in Richtung der geschlossenen Umgebung verhindert wird.

**Revendications**

1. Dispositif de passage d'air (1) pour la ventilation d'air dans des environnements fermés, comprenant :

   - un premier élément de grille (11) s'étendant principalement sur un premier plan imaginaire (A) et qui présente une première épaisseur (L1) le long d'une première direction de passage d'air (Z-Z) perpendiculaire audit premier plan imaginaire (A), où ledit premier élément de grille (11) comprend un premier corps de grille (110) dans lequel des premières ouvertures (51) sont obtenues, chacune s'étendant respectivement pour la première épaisseur (L1) totale avec une première hauteur d'ouverture (H1), le long d'axes d'ouverture principaux (C-C) qui sont sensiblement parallèles respectivement à la première direction de passage d'air (Z-Z), où lesdites premières ouvertures (51) sont délimitées par des premières parois latérales (510) ayant une première surface intérieure réfléchissante diffusive (510') ;
   - un second élément de grille (12) s'étendant sur un second plan imaginaire (B) et qui a une seconde épaisseur (L2) le long d'une seconde direction de passage d'air (Z'-Z') qui est perpendiculaire audit second plan imaginaire (B), où ledit second élément de grille (12) comprend un second corps de grille (120) dans lequel des secondes ouvertures (52) sont obtenues, chacune s'étendant respectivement pour la seconde épaisseur (L2) totale avec une seconde hauteur d'ouverture (H2), le long de seconds axes d'ouverture principaux (C'-C') qui sont sensiblement parallèles respectivement à la seconde direction de passage d'air (Z'-Z'), où lesdites secondes ouvertures (52) sont délimitées par des secondes parois latérales (520) ayant une seconde surface intérieure réfléchissante diffusive (520') ;
   - un cadre de support (15) qui supporte et relie ledit premier élément de grille (11) et ledit second élément de grille (12), le dispositif de passage d'air étant configuré de façon à ce que le premier élément de grille (11) et le second élément de grille (12) puissent être traversés en série par de l'air, le premier plan imaginaire (A) et le second plan imaginaire (B) étant mutuellement incidents avec un angle d'inclinaison (α) qui est fonction desdites première et seconde épaisseurs (L1, L2) et desdites première et seconde hauteurs d'ouverture (H1, H2), de façon à ce qu'au moins une partie d'un rayon lumineux entrant dans lesdites ouvertures (51, 52) soit absorbée par la surface intérieure réfléchissante diffusive (510', 520') d'au moins une parmi lesdites ouvertures (51, 52).

2. Dispositif de passage d'air (1) selon la revendication 1, dans lequel la première hauteur d'ouverture (H1) est inférieure à, de préférence est égale à environ la moitié de, ladite première épaisseur (L1), et la seconde hauteur d'ouverture (H2) est inférieure à, de préférence égale à environ la moitié de, ladite seconde épaisseur (L2).

3. Dispositif de passage d'air (1) selon l'une quelconque des revendications précédentes, dans lequel la première épaisseur (L1) coïncide avec la seconde épaisseur (L2).

4. Dispositif de passage d'air (1) selon l'une quelconque des revendications précédentes, comprenant au moins un élément de grille auxiliaire (16) agencé en série avec lesdits premier élément de grille (11) et second élément de grille (12).

5. Dispositif de passage d'air (1) selon l'une quelconque des revendications précédentes, dans lequel la première surface intérieure réfléchissante diffusive (510') et la seconde surface intérieure réfléchissante diffusive (520') sont faites d'un matériau ayant un coefficient de réflectance total inférieur à 0,5, de préférence inférieur à 0,2, dans le spectre de rayons lumineux d'intérêt.

6. Dispositif de passage d'air (1) selon l'une quelconque des revendications précédentes, dans lequel la première surface intérieure réfléchissante diffusive (510') et la seconde surface intérieure réfléchissante diffusive (520') sont faites d'un matériau qui réfléchit les rayons lumineux de manière diffusive.

7. Dispositif de passage d'air (1) selon l'une quelconque des revendications précédentes, comprenant un ventilateur (18) à l'intérieur du premier élément de grille (11) qui est configuré pour aspirer l'air passant à travers ledit premier élément de grille (11).

8. Système de stérilisation (9) permettant de stériliser de l'air dans un environnement fermé, comprenant :

   - une chambre d'exposition (90) s'étendant le long d'un axe (X-X), entre une extrémité d'admission d'air (91) et une extrémité d'évacuation d'air (92), où ladite chambre d'exposition (90) est délimitée de manière périphérique par un boîtier (900) ayant une surface intérieure de boîtier réfléchissante,

- une embouchure d'admission (910) à proximité de ladite extrémité d'admission d'air (91) et une embouchure d'évacuation (920) à proximité de ladite extrémité d'évacuation d'air (92) ;
- un émetteur (95) approprié pour émettre des rayons UV dans la chambre d'exposition (90) ;
- un dispositif de passage d'air (1) selon l'une quelconque des revendications précédentes ;

dans lequel ledit dispositif de passage d'air (1) est positionné à proximité de ladite embouchure d'évacuation (920) et configuré pour bloquer une sortie de rayons UV depuis le système de stérilisation (9).

9. Système de stérilisation (9) selon la revendication 8, comprenant en outre un dispositif de passage d'air (1) selon l'une quelconque des revendications 1 à 7 précédentes positionné à proximité de l'embouchure d'admission (910).

10. Système de canalisation pour environnement fermé, comprenant un dispositif de passage d'air (1) selon l'une quelconque des revendications 1 à 7, dans lequel ledit système de canalisation est configuré pour permettre la ventilation de l'environnement fermé en empêchant le passage de rayons lumineux depuis ou vers ledit environnement fermé.

FIG.1

FIG.2a

FIG.2b

EP 4 180 730 B1

FIG.3a

FIG.3b

EP 4 180 730 B1

FIG.4

FIG.5

FIG.6

FIG.7

FIG.8

EP 4 180 730 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016178698 A